# EUROPEAN PATENT APPLICATION

(11) **EP 2 465 491 A1**
(43) Date of publication of application: **20.06.2012**
(21) Application number: 12154958.8
(22) Date of filing: 18.06.2003
(51) Int. Cl.: A61K 8/97, A61Q 19/08, A61K 35/64, A61K 36/05, A61K 36/23, A61K 36/725

(54) **Anti-aging preparation**

(30) Priority: 25.06.2002 JP 2002185036
(62) Divisional of application: 03746002.9
(71) Applicant: Shiseido Company, Ltd., Chuo-ku Tokyo 104-8010 (JP)
(72) Inventor: Yano, Kiichiro, KanagawaA, 224-8558 (JP); Kajiya, Kentaro, Kanagawa, 236-8643 (JP)
(74) Representative: Salou, Clarisse

(57) **Abstract**

The present invention provides an angiogenesis inhibiting preparation and an anti-aging preparation characterized by comprising one or a plurality of crude drugs selected from the group consisting of da zao extract, ginseng extract, roman chamomile extract, chlorella extract, celery extract, parsley extract, coicis semen extract, and silk extract.

## Description

### Technical Field

The present invention relates to an angiogenesis inhibiting preparation, comprising a given agent, which exhibits TSP-1 induction activity and inhibits angiogenesis upon induction of the death of vascular endothelial cells, and an anti-aging preparation, particularly an anti-aging external preparation for skin, characterized by comprising, as an active component a given agent, and which exhibits an anti-aging action, particularly an anti-photoaging action, by virtue of an angiogenesis inhibitory activity.

### Background Art

In recent years, studies on skin aging have been made. Regarding the cause of skin aging, increasing age is, macroscopically, an important factor. In addition, the influence of drying, oxidation, sunlight (ultraviolet light) and the like may be mentioned as direct factors concerning skin aging. Specific examples of the phenomena of skin aging known in the art include a reduction in mucopolysaccharide including hyaluronic acid, a crosslinking reaction of collagen, and damage to cells by ultraviolet light.

Further, various studies on inhibition and improvement in wrinkles, fine wrinkles, loosening and the like of the skin caused by skin damage, or skin aging caused by exposure of the skin to ultraviolet light have been made, and, for example, promotion of production of hyaluronic acid (Japanese Unexamined Patent Publication (Kokai) No. 2001-163794), inhibition of production and activity of matrix metal proteinase (MMP) (Published Japanese Translation of PCT International Publication No. 2000-503660), promotion of production of collagen, and inhibition of activity of esterase (Japanese Unexamined Patent Publication (Kokai) No. 11(1999)-335235) have been elucidated to be effective. Further, as a result of such studies, it has been found that hyaluronic acid and amino acids, various polyhydric alcohols, succharides, and, further, various plant extracts are effective for inhibition and improvement in skin aging.

In these studies, attention has been drawn mainly to skin or epidermic cells for inhibition and amelioration of wrinkles and the like. Apart from them, recently, there is a report which suggests that endothelial cells in a cutaneous vascular system are also involved in aging of the skin.

An object of the present invention is to develop a novel composition that is effective for prophylaxis or inhibition of skin aging from the viewpoint of the relationship between endothelial cells in the cutaneous vascular system and skin aging.

### Disclosure of the Invention

As a result of extensive and intensive studies, the present inventors have found that some crude drugs, that is, da zao extract, ginseng extract, roman chamomile extract, chlorella extract, celery extract, parsley extract, coicis semen extract, and silk extract, inhibit angiogenesis and that, consequently, they can effectively prevent or inhibit aging.

Thus, according to the present invention, there is provided an angiogenesis inhibiting preparation characterized by comprising one or a plurality of crude drugs selected from the group consisting of da zao extract, ginseng extract, roman chamomile extract, chlorella extract, celery extract, parsley extract, coicis semen extract, and silk extract.

More specifically, according to the present invention, there is provided an anti-aging preparation characterized by comprising one or a plurality of crude drugs selected from the group consisting of da zao extract, ginseng extract, roman chamomile extract, chlorella extract, celery extract, parsley extract, coicis semen extract, and silk extract. This anti-aging preparation is very effective for prevention or inhibition of the formation of wrinkles, particularly the formation of wrinkles caused by photoaging.

Further, according to the present invention, there is provided a method for inhibiting aging, comprising the step of applying the above angiogenesis inhibiting preparation or anti-aging preparation onto the skin.

The cutaneous vascular system is essential for the maintenance of normal skin structure and function. Moreover, it plays a major role in the regulation of hair growth, in the mediation of UV-damage to the skin, in chronological skin aging, in the skin response to irritation and inflammation, and in tissue repair. While blood vessels function constantly in normal adult's skin, they respond to different stimuli with rapid activation and vessel enlargement and angiogenesis and the formation of new capillaries from preexisting blood vessels. These changes are dependent on the balance between both pro-angiogenic molecules (angiogenesis promotors) and anti-angiogenic molecules (angiogenesis inhibitors), and there is evidence that, in normal skin, a predominance of endogenous angiogenesis inhibitors over angiogenesis promotors occurs.

Recent studies suggest vascular endothelial growth factor (VEGF), also known as vascular permeability factor, as the major angiogenesis promotor in human skin. In normal skin, VEGF is secreted in small amounts by keratinocytes and binds to specific receptors on dermal microvascular endothelial cells, thereby ensuring the viability of endothelial cells and maintaining the upper vascular plexus. In epidermal hyperplasia, as in inflammation or in healing wounds, epidermal VEGF expression is highly upregulated. Accordingly, it appears that VEGF induces increased vascularization and nutritional supply in the dermis underlying the avascular epidermis.

In contrast to VEGF, two members of the thrombospondin (TSP) family of extracellular matrix proteins, that is, TSP-1 and TSP-2, play major roles as inhibitors of angiogenesis in normal skin. TSPs are expressed by several skin cells, including basal layer keratinocytes, and is deposited in the dermal-epidermal basement membrane area, contributing to the natural anti-angiogenic barrier that prevents vascularization of the epidermis. It is also known that the expression of TSP is modulated during angiogenesis and during vessel regression.

In vivo studies conducted by the present inventors, using angiogenesis inhibitor (e.g. TSP-1 and TSP-2)-knockout models, or transgenic models which overexpress an angiogenesis promotor VEGF or an angiogenesis inhibitor in the skin, suggest that modulation of the levels of these angiogenesis promotors and inhibitors markedly affects the extent of UV-damage and skin wrinkling, irritative skin responses, and the repair of skin damage.

Upon exposure of mice to ultraviolet light for a long period of time, significant wrinkles are formed. In this case, however, surprisingly, significant growth of subcutaneous blood vessels occurred together with increased vessel enlargement and branching. It was also found that exposure of mice to ultraviolet light for a long period of time increases the development of a vascular endothelial growth factor. This indicates that neogenesis of vascular endothelial cells is involved in the formation of wrinkles attributable to damage to skin caused by ultraviolet light irradiation or the like. In other words, it is apparent that the formation of wrinkles can be inhibited by inhibiting the neogenesis of vascular endothelial cells, while inducing cell death of vascular endothelial cells.

Accordingly, the present inventors have conducted screening of many crude drugs as candidate drugs for inhibiting skin aging by taking advantage of both a mechanism of angiogenesis inhibition based on induction of TSP-1 as an angiogenesis inhibitor and a mechanism of angiogenesis inhibitor based on induction of apotosis of vascular endothelial cells, and, as a result, have found the presence of several crude drugs having activity which induces TSP-1 and also induces apotosis of vascular endothelial cells. It is neither known nor suggested in the prior art at all that, among these crude drugs, da zao extract, ginseng extract, roman chamomile extract, chlorella extract, celery extract, parsley extract, coicis semen extract, and silk extract have angiogenesis inhibitory activity and antiaging activity, particularly anti-photoaging activity.

### Brief Description of the Drawings

Fig. 1 is a diagram showing the results of screening of a TSP-1 inducer; and
Fig. 2 is a diagram showing the results of screening of an apotosis inducer.

### Best Mode for Carrying Out the Invention

As described in detail in Experiment 1 of the following working example, screening of an agent capable of inducing TSP-1 as an angiogenesis inhibitor was carried out by a process comprising the steps of allowing various candidate agents to act on an HaCat cell strain transfected with a DNA construct comprising luciferase-coding gene as a reporter gene ligated to the downstream of the TSP-1 promoter, measuring the luciferase activity, regarding the luciferase activity as TSP-1 promoter activity, and then selecting an agent to activate TSP-1 promoter as an active agent.

TSP-1 inducer does not always induce the death of vascular endothelial cells. For example, fibroblast growth factor (bFGF) or tissue plasminogen activator (TPA) which is a positive control of the TSP-1 inducing substance does not induce cell death in vascular endothelial cells. Therefore, in order to identify agents which cause not only TSP-1 induction but also a TSP-1 function (i.e., death of vascular endothelial cells), screening of agents, which induce apotosis of vascular endothelial cells, was carried out as secondary screening.

Screening of agents which induce apotosis of vascular endothelial cells was carried out by a conventional method for measuring apotosis activity against cells, specifically a method in which, as described in detail in Experiment 2 of the working example described later, a candidate agent is allowed to act on vascular endothelial cells in a suitable cell culture medium, the cell is measured for cell death, and an agent capable of enhancing the death of the cells is selected as an active agent.

As a result, it was found that the following agents induce TSP-1 and further induces apotosis of vascular endothelial cells to inhibit angiogenesis.

Da zao extract: Extract of fruit of jujube. It contains, as a main component, saccharides such as saponin and fructose, organic acids, and a substance called cyclic AMP which is a nucleic acid-related substance. This cyclic AMP functions, for example, to perform neogenesis (regeneration) of sebum tissue protein and sebum secretion regulation.

Ginseng extract: Extract also known as Korean ginseng extract. It contains, as main components, ginsenoside, panaxynol, β-elemene and the like. Ginsenoside functions to promote the synthesis of protein and DNA.

Roman chamomile extract: Extract of annual grass native to Europe. It contains, as main components, terpene alcohol, chamazulene, nonylic acid and the like. A dried flower head has anti-inflammatory action and appetite stimulation action.

Chlorella extract: Extract obtained from single cell plants that inhabits fresh water. It contains, as main components, large amounts of amino acids and vitamins and has cell division activity, antitumor activity and the like.

Celery extract: Extract of annual grass native to South Europe. It contains, as main components, flavon glycoside called apiin, mannitol, bergapten, inositol and the like. Celery extract has antihypertensive action.

Parsley extract: Extract of herbaceous perennial native to Europe. It contains apiole, myristicin and the like as main components. The apiole has diuretic action.

Coicis semen extract: Extract of seed of pearl barley. It contains, as main components, amino acids such as glutamic acid, leucine, and tyrosine, and coixenolide. This coixenolide functions as an antitumor component.

Silk extract: Extract of silk fibers obtained from domesticated silkworm. It contains about 20 amino acids as main components. The efficacy of silk extract is regeneration of cells, promotion of metabolism and the like.

The above extracts can be obtained by conventional methods. For example, an extract can be obtained by either immersing a plant as an origin of each extract in an extraction solvent or heating the plant together with the extraction solvent under reflux, then conducting filtration, and concentrating the filtrate. The extraction solvent may be any solvent so far as it is usually employed in extraction. For example, water, and organic solvents, for example, alcohols such as methanol, ethanol, propylene glycol, 1,3-butylene glycol, and glycerin, hydrous alcohols, chloroform, dichloroethane, carbon tetrachloride, acetone, ethyl acetate, and hexane, may be used solely or in a combination of two or more. The extract obtained by extraction with the above solvent may be used as such, or after treatment by concentrating the extract and subjecting the concentrated extract to an adsorption method, for example, using an ion exchange resin to remove impurities, or by adsorbing the concentrated extract on a column of porous polymer (for example, Amberlite XAD-2), then eluting the extract with methanol or ethanol, and concentrating the eluate. Further, for example, extract obtained by a distribution method, for example, with water/ethyl acetate can also be used.

The above crude drugs according to the present invention are very effective for prophylaxis or inhibition of aging caused by neogenesis, growth, or proliferation of vascular endothelial cells. Aging caused by neogenesis, growth, or proliferation of vascular endothelial cells means skin damage, photoaging caused by exposure to ultraviolet light and the like. The crude drugs according to the present invention are effective, particularly for prevention and inhibition of photoaging.

Photoaging generally means a change in appearance and function of the skin observed as a result of repeated exposure to sunlight. Ultraviolet light (UV) as a constituent element of sunlight, particularly intermediate UV (called UVB, wavelength: 290 to 320 nm), mainly causes photoaging. The level of exposure to UVB necessary for causing photoaging is not known at the present time. However, repeated exposure to UVB on such a level that causes erythema or tanning generally leads to photoaging. Clinically, photoaging can be identified, for example, as rough dry skin, formation of wrinkles, colored macula, sallowing, loosening, onset of telangiectasia, formation of moles, onset of purpura, increased sensitivity to damage, occurrence of atrophic and fibrotic pigment removal region, and onset of premalignant tumor and malignant tumor. Photoaging usually occurs in skin which is habitually exposed to sunlight, such as face, ear, head, neck, and hand.

The formulating amount of the agent having angiogenesis inhibitory activity in the angiogenesis inhibiting preparation or the anti-aging preparation according to the present invention is 0.0001 to 20.0% by mass, preferably 0.0001 to 10.0% by mass, as a dry substance based on the total amount of the composition.

The angiogenesis inhibiting preparation or the anti-aging preparation according to the present invention can be properly formulated to contain, in addition to the above indispensable ingredient, optional ingredients commonly used in external preparations for skin such as cosmetics and pharmaceutical products, for example, skin-whitening agents, moisturizing agents, antioxidants, oil components, ultraviolet absorbers, surfactants, thickeners, alcohols, powder components, coloring agents, aqueous components, water, and various nutrient preparations for skin, and the like.

Further, other materials, for example, disodium edetate, trisodium edetate, sodium citrate, sodium polyphosphate, sodium metaphosphate, gluconic acid, and other sequestering agents, caffeine, tannin, verapamil, tranexamic acid and its derivatives, licorice extract, glabridin, hot-water extracts of fruits of Chinese quince, various herbicides, tocopherol acetate, glycyrrhetinic acid and its derivatives or its salts, and other drugs, vitamin C, ascorbic acid magnesium phosphate, ascorbic acid glucoside, arbutin, kojic acid, and other skin-whitening agents, glucose, fructose, mannose, sucrose, trehalose, and other saccharides, retinoic acid, retinol, retinol acetate, retinol palmitate, and other vitamin A may also be optionally formulated.

For the angiogenesis inhibiting preparation or the anti-aging preparation according to the present invention, any dosage form may be adopted without particular limitation so far as it has hitherto been used in external preparations for skin, and examples thereof include ointments, creams, milky lotions, skin lotions, packs, and bath agents.

### Examples

The following Examples further illustrate but do not limit the present invention. Formulating amounts are in % by mass.

### Experiment 1. (Primary screening)

### Screening of TSP-1 inducer

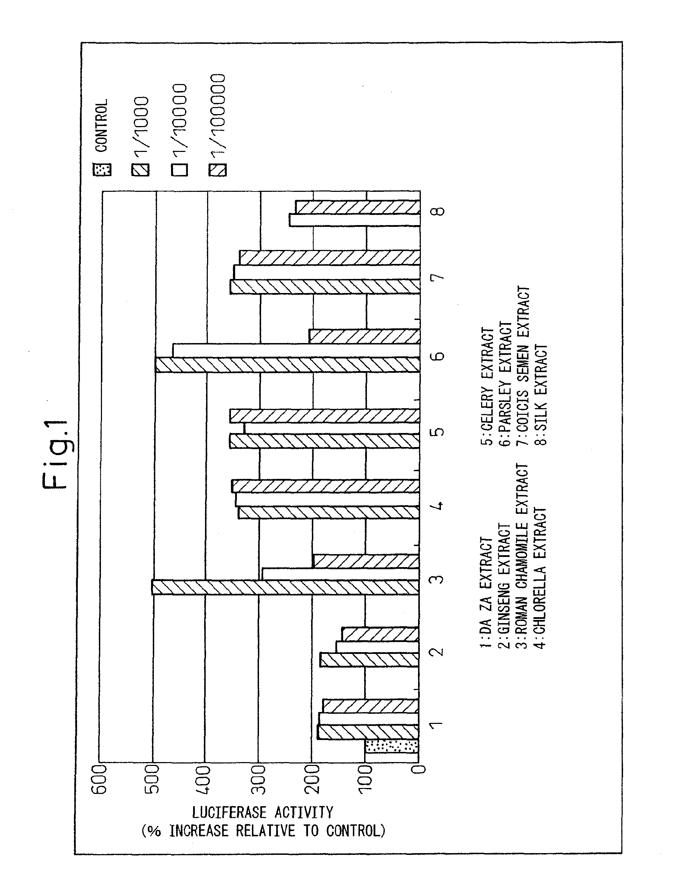

A cell strain prepared by stably transfecting HaCat cells with a DNA construct comprising a luciferase-coding gene as a reporter gene ligated to the downstream of a TSP-1 promoter, was provided. The cell strain was suspended in DMEM/F12 (Invitrogen) (containing 10% FBS and 1 mg/ml hygromycin B (Invitrogen)) medium and was seeded in a 24-well plate in an amount of 4 × 10⁴ cells per well. After about 30 hr, the medium was replaced with a serum free DMEM/F12 medium, followed by culture under a condition of 5% CO₂ 37°C for 25 hr. Thereafter, a solution of a dried product of various plant extracts as candidate agents dissolved in DMSO (Wako Pure Chemical Industries, Ltd.) to a concentration of 2% by mass was added in a volume of one thousandth, one ten thousandth, or one hundred thousandth of the culture, and culture was carried out for 24 hr. After the supernatant of the culture was removed, the cells were washed with PBS and were measured for luciferase activity with Luciferase Assay System (Promega). The luciferase activity was regarded as TSP-1 promoter activity. As a result, among the experimented various plant extracts, some extracts including da zao extract (1), ginseng extract (2), roman chamomile extract (3), chlorella extract (4), celery extract (5), parsley extract (6), coicis semen extract (7), and silk extract (8) (all extracts being available from Ichimaru Pharcos Co., Ltd.) exhibited effective TSP-1 promoter activity. The results of experiments using these crude drugs are shown in Fig. 1. For the control, DMSO was allowed to act instead of the candidate agents.

As described above, the TSP-1 inducer does not necessarily induce the death of vascular endothelial cells. Accordingly, the inventors have further carried out secondary screening for screening of agents capable of exhibiting not only TSP-1 induction but also TSP-1 function (i.e. cell death of vascular endothelial cells).

### Experiment 2. (Secondary screening)

### Screening of apotosis inducer

The supernatant of the culture after culture for 24 hr after addition of the agent (one thousandth) in the primary screening was added to vascular endothelial cell HMVEC (Sanko Junyaku Co., Ltd.) which had been cultured on a chamber slide until about 70% confluent, followed by culture for 24 hr to examine cell death induction of vascular endothelial cells. The detection of the cell death was carried out by using an ApopTag Plus Fluorescein In Situ Apoptosis Detection Kit, and calculation of the cell death induction ratio was carried out by calculating the proportion of the number of the died cells in the group to which the agent had been added, relative to the that of the control group. The calculation was carried out by a process comprising taking images for three sites in each slide of the group in which the agent had been added and the control group by a digital camera, and then calculating the proportion of the number of cell death-signals to the number of nuclei in each image (%).

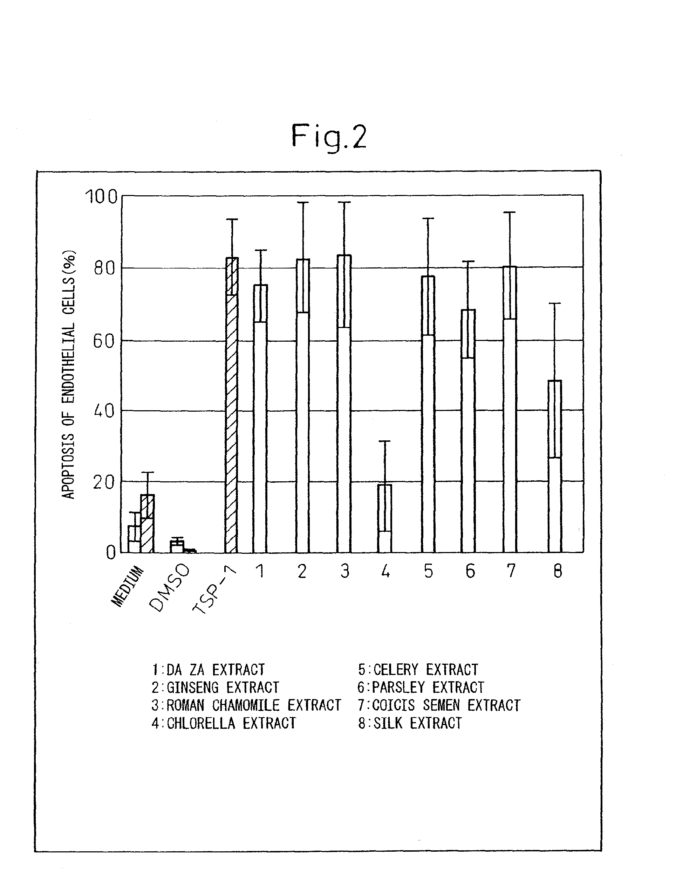

The results revealed that, among the tested various plant extracts, da zao extract (1), ginseng extract (2), roman chamomile extract (3), chlorella extract (4), celery extract (5), parsley extract (6), coicis semen extract (7), and silk extract (8) effectively induce apotosis of vascular endothelial cells. The results of the experiment using these crude drugs are shown in Fig. 2. For comparison, the results of an experiment in which only the medium (in the drawing, right bar: one ten thousandth, left bar: one thousandth), DMSO (in the drawing, right bar: one ten thousandth, left bar: one thousandth), or TSP-1 (one thousandth) (Haematologic Technologies, Inc.) was allowed to act instead of the candidate agents are also shown.

The results of the above two types of screening show that da zao extract, ginseng extract, roman chamomile extract, chlorella extract, celery extract, parsley extract, coicis semen extract, and silk extract are effective for inducing TSP-1 and inducing apotosis of vascular endothelial cells.

### Industrial Applicability

Agents, which induce TSP-1 and induce apotosis of vascular endothelial cells, such as da zao extract, ginseng extract, roman chamomile extract, chlorella extract, celery extract, parsley extract, coicis semen extract, and silk extract, are effective for inhibition of aging.

## Claims

1. A composition comprising one or a plurality of agents capable of inducing TSP-1 induction and further inducing apoptosis of vascular endothelial cells to inhibit angiogenesis selected from the group consisting of da zao (*Zizyphus jujuba Miller var. inermis Rehder*) extract, celery (*Aplum graveolens Linne*) extract and silk (*Bombyx mori Linnaeus*) extract, for the prevention or inhibition of wrinkles caused by photoaging.

2. Cosmetic use of one or a plurality of agents capable of inducing TSP-1 induction and further inducing apoptosis of vascular endothelial cells to inhibit angiogenesis selected from the group consisting of da zao (*Zizyphus jujuba Miller var.inermis Rehder*) extract, celery (*Aplum graveolens Linne*) extract and silk (*Bombyx mori Linnaeus*) extract, for preventing or inhibiting wrinkles caused by photoaging.
